(19) Europäisches Patentamt

European Patent Office

Office européen des brevets

(11) **EP 4 624 629 A1**

(12) **EUROPEAN PATENT APPLICATION**

(43) Date of publication:
**01.10.2025 Bulletin 2025/40**

(21) Application number: **24166461.4**

(22) Date of filing: **26.03.2024**

(51) International Patent Classification (IPC):
*C23F 13/02* (2006.01) *C23F 13/06* (2006.01)

(52) Cooperative Patent Classification (CPC):
**C23F 13/06; C23F 13/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Volvo Penta Corporation**
**405 08 Göteborg (SE)**

(72) Inventors:
• **RÄFTEGÅRD, Viktor**
**448 31 FLODA (SE)**
• **KÄLLVIK, Johan**
**441 74 SOLLEBRUNN (SE)**

(74) Representative: **Kransell & Wennborg KB**
**P.O. Box 2096**
**403 12 Göteborg (SE)**

(54) **MARINE SALINITY MEASURING ARRANGEMENT AND METHOD**

(57) In a first aspect, an arrangement for measuring salinity in water is provided, which arrangement is part of an impressed current cathodic protection (ICCP) system where the resistance of formed by a reference electrode (324) and another electrode is determined. The resistivity of the water is then calculated based on the determined resistance and at least one stored electrode property value, which resistivity is inversely proportional to the salinity.

Fig. 6

EP 4 624 629 A1

**Description**

TECHNICAL FIELD

The present disclosure generally relates to a salinity measuring arrangement for marine objects. In particular aspects, the arrangement utilizes an on-board impressed current cathodic protection system. The disclosure may also relate to a marine object with such an arrangement, and also to a method for operating such an arrangement.

BACKGROUND

Seawater is a corrosive environment and the parts used for marine propulsion units and other immersed metallic parts require some form of cathodic protection in order to eliminate or reduce corrosion of those parts. An efficient way of providing corrosion protection is the use of a method termed impressed current cathodic protection (ICCP). ICCP systems are often used on cargo carrying ships, tankers and larger pleasure craft. According to a general principle for an ICCP system, a metal element and an anode are attached to a vessel and immersed in water. The metal element is connected to the negative terminal and the anode is connected to the positive terminal of a source DC electrical power to provide an electric de-passivation current through an electrical circuit including the anode, the metal element and the electrolyte. In this way, the anode provides corrosion protection for the metal parts. By maintaining a predetermined potential in the electrical circuit, the ICCP system can provide a desired protection level for the metal parts to be protected.

Marine vessels moving in waterways such as river estuaries will be exposed to seawater, fresh water and brackish water comprising a mixture of these. With reduced salinity the resistivity of the water in which the vessel is immersed increases. At some point the electrical resistance in the electrical circuit of the corrosion protection system may increase to a level where the ICCP is unable to maintain the potential of the protected structure within an acceptable interval. An ICCP system may interpret the reduced potential and the corresponding low protection level as an internal error or a malfunction caused by external factors. When this occurs, the ICCP system may shut down and switch to passive backup galvanic anodes for protection. In fresh or brackish water galvanic anodes will provide little or no corrosion protection.

The disclosure provides an improved impressed current corrosion protection system aiming to determine degree of salinity in the water.

SUMMARY

According to a first aspect of the disclosure, an arrangement for measuring salinity in water is provided, which arrangement is part of an impressed current cathodic protection (ICCP) system having an electrical circuit comprising a control unit for controlling the impressed current cathodic protection system, a first direct current power source, a plurality of electrodes including at least one active anode electrode connectable to a positive terminal of the first power source, at least one cathode electrode connectable to a negative terminal of the first power source and at least one reference electrode connectable to said cathode electrode for measuring an electrical potential of the cathode electrode with the reference electrode as a ground reference, the electrical potential being indicative of the surface polarization of the water at the cathode electrode, and the control unit being configured to control the power supplied by the first direct current power source based on the measured electrical potential; the electrical circuit further comprising a second power source, a first terminal to which the at least one reference electrode is connectable, a voltage sensor detecting an output voltage impressed on the reference electrode upon the reference electrode being connected to the second source of electric power, a current sensor detecting a current supplied to the reference electrode upon the reference electrode being connected to the second source of electric power, wherein the control unit is configured to initiate a measurement sequence at determined intervals, during which measurement sequence the control unit is configured to disconnect the reference electrode from the cathode electrode, connect the reference electrode to the first terminal of the second power source, connect at least another one of the electrodes to a second terminal of the second power source while disconnecting said another electrode from the first direct current power source, register said output voltage, register said current, determine the resistance of the circuit formed by the reference electrode and said another one of the electrodes using the output voltage and the current, and calculate the resistivity of the water based on the determined circuit resistance and at least one stored electrode property value, which resistivity is inversely proportional to the salinity. The first aspect of the disclosure may seek to resolve an issue of measuring salinity in water for a marine object. A technical benefit may include to improve accuracy of the salinity measurement.

Optionally in an example, said at least another one of the electrodes is the cathode electrode.

Optionally in an example, said at least another one of the electrodes is the active anode electrode.

Optionally in an example, the at least one reference electrode comprises at least two reference electrodes each individually connectable to said cathode electrode, said at least another one of the electrodes being a second one of the at least two reference electrodes.

Optionally in an example, the plurality of electrodes further includes at least one passive anode electrode connectable to the positive terminal of the first direct current power source, the passive anode electrode serving as a sacrificial electrode for back-up protection, the control unit being configured to connect the passive anode electrode to the positive terminal of the first direct current power source, while disconnecting the active anode electrode from the positive terminal of the first direct current power source, to form a circuit with the cathode electrode to attain back-up protection.

Optionally in an example, the plurality of electrodes further includes at least one passive anode electrode connectable to the positive terminal of the first direct current power source, the passive anode electrode serving as a sacrificial electrode for back-up protection, the control unit being configured to connect the passive anode electrode to the positive terminal of the first direct current power source, while disconnecting the active anode electrode from the positive terminal of the first direct current power source, to form a circuit with the cathode electrode to attain back-up protection, said at least another one of the electrodes being the passive anode electrode.

Optionally in an example, the second power source is an alternative current power source or a direct current power source;

Optionally in an example, the control unit is configured to disconnect the active anode electrode from the first direct current power source upon said another of the electrodes being connected to the second power source.

Optionally in an example, the at least one stored electrode property value is the surface area of said at least another one of the electrodes and/or the surface area of the reference electrode.

Optionally in an example, the control unit is configured to maintain the impressed current cathodic protection system in operation if the determined resistivity is above a set threshold value.

Optionally in an example, the control unit is configured to determine a current salinity value based on the determined resistivity and to generate an output signal indicating the salinity value to a user.

Optionally in an example, the control unit is configured to monitor changes in the determined resistivity; to compare an increase in the determined resistivity to stored values for resistivity; and to determine if the increase is indicative of an electrical circuit malfunction.

Optionally in an example, a marine object is provided with an impressed current cathodic protection system comprising an arrangement for measuring salinity according to the first aspect.

According to a second aspect of the disclosure, a method for measuring salinity in water is provided using an arrangement being part of an ICCP system having an electrical circuit comprising a control unit for controlling the impressed current cathodic protection system, a first direct current power source, a plurality of electrodes including at least one active anode electrode connectable to a positive terminal of the first power source, at least one cathode electrode connectable to a negative terminal of the first power source and at least one reference electrode connectable to said cathode electrode for measuring an electrical potential of the cathode electrode with the reference electrode as a ground reference, the electrical potential being indicative of the surface polarization of the water at the cathode electrode, and the control unit being configured to control the power supplied by the first direct current power source based on the measured electrical potential; the electrical circuit further comprising a second power source, a first terminal to which the at least one reference electrode is connectable, a voltage sensor detecting an output voltage impressed on the reference electrode upon the reference electrode being connected to the second source of electric power, a current sensor detecting a current supplied to the reference electrode upon the reference electrode being connected to the second source of electric power, wherein the control unit is configured to initiate a measurement sequence at determined intervals, during which measurement sequence the control unit is configured to disconnect the reference electrode from the cathode electrode, connect the reference electrode to the first terminal of the second power source, connect at least another one of the electrodes to a second terminal of the second power source while disconnecting said another electrode from the first direct current power source, register said output voltage, register said current, determine the resistance of the circuit formed by the reference electrode and said another one of the electrodes using the output voltage and the current, and calculate the resistivity of the water based on the determined circuit resistance and at least one stored electrode property value, which resistivity is inversely proportional to the salinity. The second aspect of the disclosure may seek to resolve an issue of measuring salinity in water for a marine object. A technical benefit may include to improve accuracy of the salinity measurement.

In some examples, a computer program product is provided comprising program code for performing, when executed by the control circuit, the method of the second aspect.

In some examples, a non-transitory computer-readable storage medium is provided comprising instructions, which when executed by the control circuit, cause the arrangement to perform the method of the second aspect.

The above aspects, accompanying claims, and/or examples disclosed herein above and later below may be suitably combined with each other as would be apparent to anyone of ordinary skill in the art.

Additional features and advantages are disclosed in the following description, claims, and drawings, and in part will be readily apparent therefrom to those skilled in the art or recognized by practicing the disclosure as described herein. There are also disclosed herein control units, computer readable media, and computer program products associated with the above discussed technical benefits.

BRIEF DESCRIPTION OF THE DRAWINGS

Examples are described in more detail below with reference to the appended drawings.

Fig. 1  shows a schematically illustrated vessel comprising a marine corrosion protection system according to an example;

Fig. 2  shows a schematic a cross-section of the rear portion of the marine vessel;

Fig. 3A  shows a schematic representation of an electrical circuit for the corrosion protection system of the vessel in Figure 2 when in active corrosion protection mode;

Fig. 3B  shows the electrical circuit of Figure 3A when in salinity measurement mode performing a salinity measurement between cathode and reference electrode;

Fig. 3C  shows a flowchart of a method of performing a salinity measurement according to an example;

Fig. 3D  shows the electrical circuit of Figure 3A when in passive corrosion protection mode, the circuit further comprising a passive anode;

Fig. 4  shows the electrical circuit of Figure 3C when in salinity measurement mode performing a salinity measurement between passive and reference electrode;

Fig. 5  shows the electrical circuit of Figure 3A when in salinity measurement mode performing a salinity measurement between active anode and reference electrode; and

Fig. 6  shows the electrical circuit of Figure 3A when in salinity measurement mode performing a salinity measurement between a first reference electrode and a second reference electrode.

DETAILED DESCRIPTION

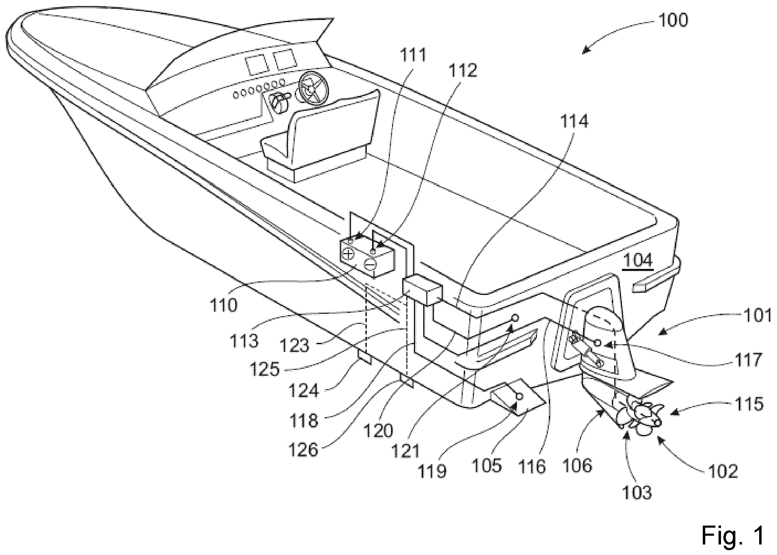

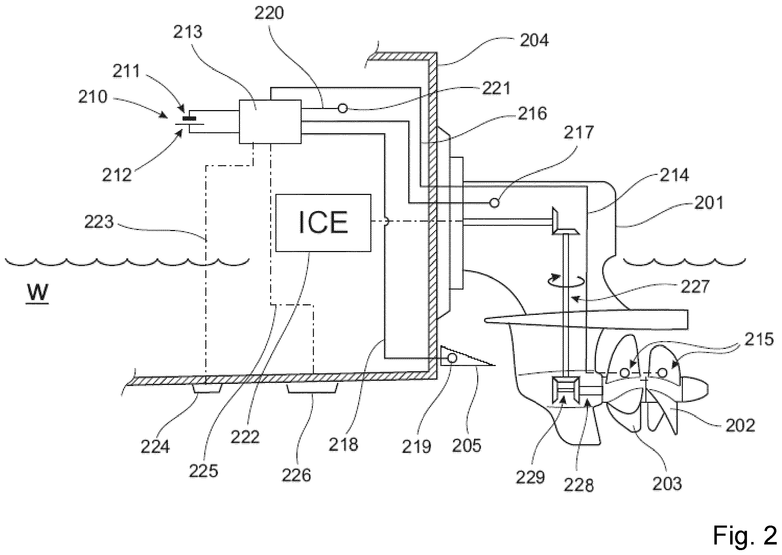

Figure 1 shows a schematically illustrated marine vessel 100 comprising a corrosion protection arrangement. According to examples, this arrangement is adapted to provide for measuring of salinity in water. As mentioned, the example discussed herein may be applied to various marine objects, such as boats, docks, bridges, rafts, etc. The vessel 100 comprises a hull with a transom 104 to which a marine propulsion system is attached. The propulsion system in this example comprises a single driveline housing 101 at least partially submerged in water, a torque transmitting drive shaft 106 extending out of the driveline housing 101, and a pair of counter-rotating propellers 102, 103 mounted on the drive shaft 106. In the current example, both propellers 102, 103 are electrically isolated from its drive shaft 106. The drive shaft arrangement is shown in Figure 2 and will be described in further detail below. Each metallic component 101, 104, 105 to be protected against corrosion is connected to a negative terminal 112 of a direct current (DC) power source 110, such as a battery, in order to form cathodes. A control unit 113 is connected to the direct current power source 110 and distributes current to all component parts forming an electrical circuit. The control unit 113 is arranged to regulate the voltage and current output from the direct current power source 110. In order to assist regulation of the voltage and current output a reference electrode 124 is utilized and connected to the control unit 113 via an electrical wire 123. The reference electrode 124 measures a voltage difference between itself and the metallic components, which is directly related to the amount of protection received by the anode. The control unit 113 compares the voltage difference produced by the reference electrode 124 with a pre-set internal voltage. The output is then automatically adjusted to maintain the electrode voltage equal to the pre-set voltage.

The at least one metallic component to be protected forms a cathode and can be the at least one driveline housing, at least one trim tab, seawater intake, swimming platform and/or at least a portion of the vessel hull. Note that this is a non-exclusive list of metallic components suitable for corrosion protection. At the same time, the ICCP arrangement provides marine growth protection for the at least one anode.

Regulation of the voltage and current output from the direct current power source is controlled to automate the current output while the voltage output is varied, or to automate the voltage output while the current output is varied. This allows the corrosion protection level to be maintained under changing conditions, e.g. variations in water resistivity or water velocity. In a sacrificial anode system, increases in the seawater resistivity can cause a decrease in the anode output and a decrease in the amount of protection provided, while a change from stagnant conditions results in an increase in current demand to maintain the required protection level. With ICCP systems, protection does not decrease in the range of standard seawater nor does it change due to moderate variations in current demand. An advantage of ICCP systems is that they can provide constant monitoring of the electrical potential at the water/hull interface and can adjust the output voltage to the anodes in relation to this. An ICCP system comprising a reference electrode is more effective and reliable than sacrificial anode systems where the level of protection is unknown and uncontrollable.

The corrosion protection arrangement is an impressed current cathodic protection (ICCP) arrangement using the propellers 102, 103 as an anode 115. In Figure 1, the metallic component to be protected against corrosion is the driveline housing 101, the trim tabs 105 (one shown), and a metal portion of the hull, in this case the transom 104. Note that this is a non-exclusive list of metallic components suitable for corrosion protection. In order to achieve this, the positive terminal 111 and the negative terminal 112 of the battery 110 are connected to the control unit 113. The control unit 113 is arranged to connect the positive terminal 111 to the propellers 102, 103 via a first electrical wire 114. The control unit 113 is further arranged to connect the negative terminal 112 to an electrical connector 117 on the driveline housing 101 via a second electrical wire 116. The negative terminal 112 is also connected to an electrical connector 119 on the trim tab 105 via a third electrical wire 118, and connected to an electrical connector 121 on the transom 104 via a fourth electrical wire 120. The corrosion protection arrangement may further optionally be provided with a passive, sacrificial anode 126 that can provide protection if a failure occurs in the active corrosion protection arrangement. The sacrificial anode 126 can be located at any suitable location on the vessel and is connectable to the control unit 113 via a fifth electrical wire 125. According to the examples, the control unit 113 is further adapted to operate as an arrangement for measuring salinity in water. The arrangement for measuring salinity will be described in detail below.

Figure 2 shows a cross-section of the rear portion of the marine vessel 100 of Figure 1, through a transom 204 and a driveline housing 201. The single driveline housing 201 is partially submerged in water and comprises torque transmitting drive shafts 228, 229 extending out of the driveline housing 201. A pair of counter-rotating propellers 202, 203 is mounted on their respective drive shafts 229, 228. In this example, the drive shafts 228, 229 are driven by an internal combustion engine (ICE) 222 via a transmission 227. Transmissions for driving counter-rotating propellers are well known in the art and will not be described in detail here. Alternative drive units for driving the propellers are possible within the scope of the disclosure. For instance, drive units comprising one or more pushing or pulling propellers can be used within the scope of the disclosure. Although the described examples relate to drive units mounted on a transom, the disclosure can be applied to most drive installations, such as outboard/inboard installations, Z-drives and azimuthing pod installations. The disclosure is not dependent on the type of power source provided, but can be applied to marine vessels using ICE, hybrid or electric power sources for propulsion or power generation.

In the example shown in Figures 1 and 2, the at least one propeller is used as an anode, wherein the at least one propeller is electrically isolated from its respective drive shaft. This is not a requirement for examples where the propeller is not used as an anode. In the current example, both propellers 202, 203 are electrically isolated from its respective drive shaft 228, 229. The propellers are electrically isolated from their respective drive shafts by a torque transmitting electrically isolating component mounted between a propeller and its respective drive shaft. The electrically isolating component is mounted in a gap formed by the outer surface of the drive shaft and the inner surface of the propeller hub. The torque transmitting electrically isolating component can be made from an elastic material, such as a natural or synthetic rubber. The propellers are made from an inert anode material, such as titanium, niobium or a similar suitable metal or metal alloy. A dielectric shield can be provided on the drive shaft between each propeller hub and the drive shaft on which the propeller is mounted. A non-exclusive list of suitable materials for use in such a dielectric shield includes polymer or polymer-ceramic materials with suitable dielectric properties.

As schematically indicated in Figure 2, each electrically isolated propeller 202, 203 is connected to a positive terminal 211 of a direct current power source 210 at schematically indicated points 215 via electrical wiring 214. The electrical connection of the propellers will be described in further detail below. Further, each metallic component 201, 204, 205 to be protected against corrosion is connected to a negative terminal 212 of the direct current power source 210. A control unit 213 is arranged to regulate the voltage and current output from the direct current power source 210. As described above, the positive terminal 211 and the negative terminal 212 of the battery 210 are connected to the control unit 213. The control unit 213 is arranged to connect the positive terminal 211 to the propellers 202, 203 via a first electrical wire 214. The control unit 213 is further arranged to connect the negative terminal 212 to an electrical connector 217 on the driveline housing 201 via a second electrical wire 216. The negative terminal 212 is also connected to an electrical connector 219 on the trim tab 205 (one shown) via a third electrical wire 218, and connected to an electrical connector 221 on the transom 204 via a fourth electrical wire 220. A reference electrode 224 is mounted on the hull remote from the propellers 202, 203 forming an anode and connected to the control unit 213 via an electrical wire 223. Regulation of the voltage and current output from the direct current power source using the control unit 213 has been described above. As discussed hereinabove, the ICCP arrangement may optionally further be provided with a passive, sacrificial anode 226 that can provide protection if a failure occurs in the active ICCP arrangement. The sacrificial anode 226 can be located at any suitable location on the vessel and is connectable to the control unit 213 via a fifth electrical wire 225. The corrosion protection systems described in Figures 1 and 2 are examples of such arrangements that can be adapted for salinity measurement, as will be described below.

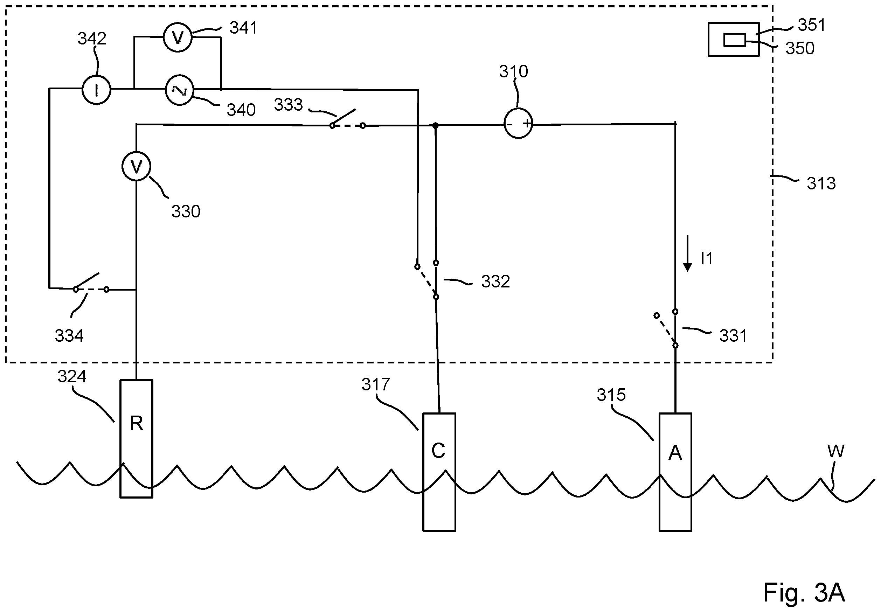

Figure 3A shows a schematic first representation of an electrical circuit for the corrosion protection system of the vessel in Figure 2 in its normal, active operating mode. A first DC power source in the form of a battery 310 is connected to, and adapted to provide electrical power to an active anode 315 (A) and at least one cathode 317 (C) to be protected. This connection is provided via a control unit 313, which is adapted to vary and control the electrical power to the active anode 315 and the cathode 317, as supplied by the battery 310.

The control unit 313 may be embodied by processing circuitry in the form of e.g. one or more microprocessors arranged to execute a computer program 350 downloaded to a storage medium 351 associated with the microprocessor, such as a Random Access Memory (RAM), a Flash memory or a hard disk drive. The processing circuitry is arranged to cause the arrangement to perform desired operations when the appropriate computer program 350 comprising computer-executable instructions is downloaded to the storage medium 351 and executed by the processing circuitry. The storage medium 351 may also be a computer program product comprising the computer program 350. Alternatively, the computer program 350 may be transferred to the storage medium 351 by means of a suitable computer program product, such as a Digital Versatile Disc (DVD) or a memory stick. As a further alternative, the computer program 350 may be downloaded to the storage medium 351 over a network. The processing circuitry may alternatively be embodied in the form of a digital signal processor (DSP), an application specific integrated circuit (ASIC), a field-programmable gate array (FPGA), a complex programmable logic device (CPLD), etc.

The control unit 313 is adapted to measure an electrical potential of the cathode 317 with a reference electrode 324 (R) as a ground reference. While a single reference electrode 324 is shown in Figure 3A, a plurality of reference electrodes may advantageously be utilized in order to provide redundancy, in case one or more reference electrodes would fail. This will be discussed in more detail subsequently. The electrical potential of the cathode 317 is measured using a voltage sensor 330. The electrical potential is indicative of the surface polarization at the interface between the cathode 317 and an electrolyte W; in this case water. The control unit 313 is further adapted to control the electrical power to the active anode 315 (A) and the cathode 317 (C) based at least partly on the measured electrical potential of the cathode 317 with the reference electrode 324 (R) as a ground reference. Through the control of the electrical power, a first electrical current (indicated in Fig.3A with an arrow 11), through an electrical circuit comprising the active anode 315, the cathode 317 and the electrolyte W, is controlled. As will be described, switch 333 is temporarily closed each time a reading of the voltage sensor 330 is to be undertaken (e.g. each 10 seconds) and open at all other occasions.

More specifically, the parameter of interest for control of the corrosion protection of the cathode 317 is the electrical potential of the cathode 317 with the reference electrode 324 as a ground reference, corresponding to the surface polarization at the interface between the cathode 317 and the water W, and the electrical power to the active anode 315 and the cathode 317 is subjected to a closed loop control so as for said surface polarization to assume a desired value. Thus, the control unit 313 will continuously control the voltage of the power source 310 to assume the desired value (which typically varies depending on e.g. implementation specifics and/or type of vessel)

Thus, the corrosion protection system for the cathode 317 comprises an ICCP system with the active anode 315, the reference electrode 324, the battery 310 and the control unit 313. In Figure 3A the schematic electrical circuit of the corrosion protection system is only shown to comprise a single cathode, in this case the drive 317. However, additional components to be protected, such as the trim tabs, the transom and other metallic components (see Fig.2) can be connected to the control unit 313 as cathodes in the same way as the drive 317.

The control unit 313 further comprises a number of controllable switches for controlling different functions of the corrosion protection system. A first switch 331 is arranged between the positive terminal of the battery 310 and the anode 315, which first switch 331 is normally closed to supply the anode with power during an active corrosion protection mode. When opened, the first switch 331 disconnects the active anode 315 from the positive terminal of the battery 310. A second switch 332 is arranged between the negative terminal of the battery 310 and the cathode 317, which second switch 332 is normally switched to a closed position to maintain a closed circuit including the active anode 315, the cathode 317 and the battery 310 during active corrosion protection mode, wherein a current $I_1$ flows from the battery 310 to the active anode 315. When opened, the second switch 332 can disconnect the cathode 317 from the negative terminal of the battery 310.

A third switch 333 is arranged between the negative terminal of the battery 310 and the reference electrode 324 (and the voltage sensor 330). Thus, each time the control unit 313 measures the electrical potential of the cathode 317 with the reference electrode 324 as a ground reference (i.e. by reading voltage sensor 330), the second switch 331 is opened and the third switch 333 is closed. The control unit 313 may hence subsequently control the voltage supplied by the power source 310 for the surface polarization as measured by the voltage sensor 330 to assume said desired value.

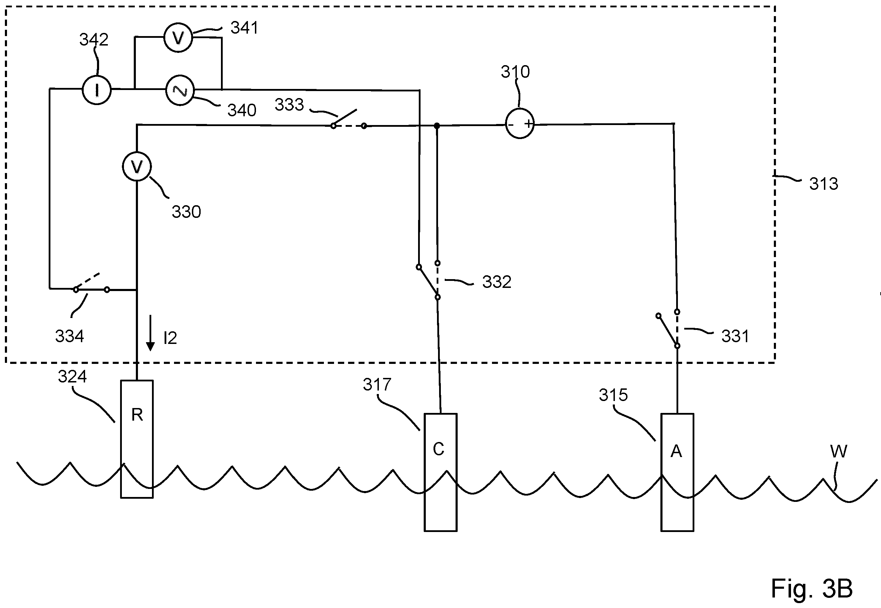

Figure 3B shows a schematic second representation of the corrosion protection system of the vessel in Figure 2 in a salinity measurement mode. Figure 3B describes a first out of a plurality of examples for implementing the salinity measurement mode where the salinity measurement is undertaken between the cathode 317 and the reference electrode 324.

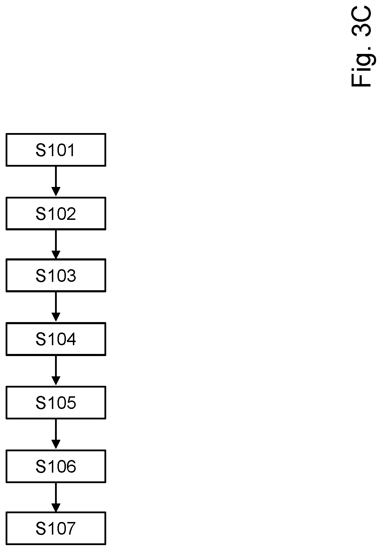

Reference will further be made to Figure 3C showing a flowchart of a method of performing a salinity measurement according to an example.

There are a number of advantages of using a reference electrode for salinity measurements:

- The reference electrode (as well as the active anode) has a known and constant surface area unlike the cathode and passive anode.
- The reference electrode is not energized during normal operation, i.e. the reference electrode is not polarized but kept at its open circuit potential. The expected voltage-current behaviour of the reference electrode is therefore quite predictable.
- Ag/AgCl electrodes may be used and have the advantage that they do not polarize easily, which means that such electrodes will return to the open circuit potential quickly after being energized and polarization is not likely to impose a significant impact on the measurements.

However, in the salinity measurement mode, the switches in the electrical circuit are controlled by the control unit 313 so that after the third switch 333 is opened in S101 (after a voltage reading is made as discussed above) for disconnecting the reference electrode 324 from the cathode 317, the fourth switch 334 is closed for connecting the reference electrode 324 to a second power source 340 in S102 and the cathode 317 connects in S103 via the second switch 332 to the second power source 340 and further via the fourth switch 334 to the reference electrode 324. The cathode 317 is thus disconnected from the first DC power source 310 in S103. At this stage, since the third switch 333 is open, the first switch 331 may either be opened or closed. The second power source 340 is illustrated as an alternating current (AC) power source, although a DC power source may be utilized. In examples to be described in the following, the second power source 340 will be illustrated in the form of an AC power source. Thus, a current I2 will flow through the path formed by the cathode 317, the second switch 332, the fourth swich 334, the reference electrode 324 and the water W.

Using an AC power source is advantageous since the usage of a DC source causes the electrodes to be polarized and creates a potential drop in the electrolyte between the electrodes, which influences the measurements. Further, when transmitting a current through Ag/AgCl electrodes in an electrolyte containing chloride ions, the AgCl layer may build on the anode and be consumed on the cathode. This is avoided by applying a high frequency AC signal.

The control unit 313 is arranged to interrupt the corrosion protection mode and switch to the salinity measurement mode at regular intervals to monitor the salinity of the water in which the vessel is operated. Any suitable time interval can be selected for this purpose, although an interval of 5-10 minutes is sufficient for the intended purpose.

During the measurement sequence of Figure 3B, the control unit 313 is arranged to register in S104 the output voltage supplied by the second power source 340 to the measurement circuit formed between the cathode 317 and the reference electrode 324 using a voltage sensor 341. The control unit 313 is further arranged to register in S105 the current using a current sensor 342. Subsequently, the circuit resistance can be determined in S106 using the output voltage and the current, by applying Ohm's law. Based on the determined circuit resistance and at least one stored electrode property value, the resistivity of the electrolyte can be calculated in S107. In this example, the stored electrode property values of the surface area $A_c$ of the cathode 317 is used. Alternatively, the surface area $A_c$ of the cathode 317 and the surface area $A_r$ of the reference electrode 324 can be used.

According to one example, the control unit is arranged to calculate the resistivity ($\rho$) using equation (1):

$$R_c = k * \rho * (\frac{1}{\sqrt{A_c}})$$

wherein:

$R_c$ is the circuit resistance ($\Omega$);
$k$ is a correlation factor (-);
$\rho$ is the resistivity of the electrolyte ($\Omega$cm);
$A_c$ is the surface area of the cathode (cm$^2$).

In this example, only the surface area $A_c$ of the cathode 317 is used. This formula can be used if the surface area $A_r$ of the reference electrode 324 is relatively large, whereby the contribution of this surface area is negligible.

According to another example, the control unit is arranged to calculate the resistivity ($\rho$) using equation (2):

$$R_c = k * \rho * (\frac{1}{\sqrt{A_c}} + \frac{1}{\sqrt{A_r}})$$

wherein:

$R_c$ is the circuit resistance ($\Omega$);
$k$ is a correlation factor (-);
$\rho$ is the resistivity of the electrolyte ($\Omega$cm);
$A_c$ is the surface area of the cathode (cm$^2$);
$A_r$ is the surface area of the reference electrode (cm$^2$).

In this example both, the surface area $A_c$ of the cathode 317 and the surface area $A_r$ of the reference electrode 324 is used.

As the resistivity is inversely proportional to the salinity, a current value for electrolyte salinity can be obtained using a stored conversion table and stored in a memory. Stored salinity values can subsequently be retrieved for comparison with updated salinity values.

A standard value for the correlation factor k can be taken from the McCoy formula:

$$R_c = 0{,}315 * \rho * \left(\frac{1}{\sqrt{A_c}}\right)$$

wherein the correlation factor k=0,315 is a standard applicable to electrode that are flush mounted onto a hull or a similar surface. Electrodes can have different shapes and sizes, depending on design, which will affect the surface area and thereby the correlation factor. The correlation factor can also be dependent on which component part, e.g. a propeller, that is used as an active anode in the corrosion protection mode. Consequently the value of the correlation factor can vary. Suitable values for the correlation factor can be determined by testing and calibration of each system or type of installation. The surface area can also vary depending on the amount of surface oxidation or whether one or more components have been fully or partially coated with an anti-corrosion coating subsequent to the installation of the system.

As long as the detected salinity has a value within a range representing normal variations for sea water, having approximately 3,5 % salinity, the corrosion protection system resumes normal operation after exiting the salinity measurement mode. The detected salinity values are registered for comparison with subsequently detected values. When a decrease in salinity is detected, the control unit 313 will automatically attempt to compensate for this by regulating the voltage supplied by the DC power source 310 to maintain a desired potential. If the detected salinity value drops to a value at or near zero, the control unit 313 will no longer be able to compensate for this to maintain the desired potential. However, by comparing a currently detected salinity value with previously registered values, the control unit 313 can determine that the reduction of the salinity value is caused by the vessel moving into a body of fresh or brackish water. By making this determination, the control unit can establish that the inability to compensate for the drop in potential is caused by a change in salinity value and not by a malfunction in the corrosion protection system. Consequently, the corrosion protection system will continue to operate, albeit at reduced efficiency level. It may be envisaged that a different criteria is used in freshwater where the corrosion risk is lower. This further lowers the risk of having to e.g. send an alert to the boat operator as a result of the malfunction.

The control unit 313 can thus be arranged to determine a current salinity value based on the determined resistivity and to generate an output signal indicating the salinity value to a user. This can advantageously be used to alert the user to changes in salinity, which changes can cause error messages to be generated by the ICCP system. The user can then choose to ignore such error messages, knowing that the cause is changes in salinity, or to monitor and possibly intervene in the operation of the ICCP system to ensure that corrosion protection is maintained.

The control unit 313 can at the same time be arranged to monitor changes in the determined resistivity; to compare an increase in the determined resistivity to stored values for resistivity; and to determine if the increase is indicative of an electrical circuit malfunction. Depending on recorded changes in resistivity over time, the control unit can either decide that the increase is indicative of an increase in salinity or that the increase is indicative of an electrical circuit malfunction. In the former case the ICCP operation may be maintained, while the latter case may cause the control unit to terminate ICCP operation in favour of passive corrosion protection.

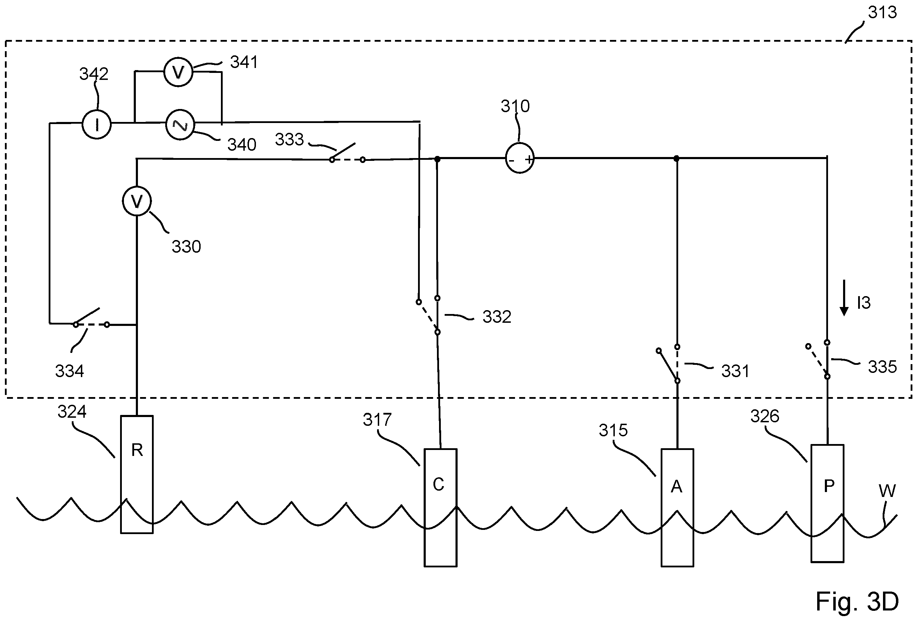

As previously mentioned, in an example a sacrificial, or passive, anode 326 (P) may be connected/disconnected to/from the corrosion protection system to provide passive corrosion protection. Such an example is illustrated in Figure 3D.

As shown, the passive anode 326 is connected/disconnected to/from the corrosion protection system by means of fifth switch 335 connecting the passive anode to the positive terminal of the DC power source 310 (possibly via a not shown variable resistor). The fifth switch 335 is normally open - thereby disconnecting the passive anode 326 from the corrosion protection system - in order to attain either the active corrosion protection mode of Figure 3A or the salinity measurement mode of Figure 3.

However, when switching from the active corrosion protection mode of Figure 3A to the passive corrosion protection mode of Figure 3D, the first switch 331 is opened to disconnect the active anode 315 and the fifth switch 335 is closed to connect the passive anode 326 to the system (while the second switch 332 remains closed for connecting the cathode 317 to the passive anode 326 via the DC power source 310). Thus, a current I3 will flow through the path formed by the cathode 317, the second switch 332, the fifth switch 335, the passive anode 326 and the water W.

The corrosion protection system for the cathode 317 thus comprises a passive corrosion protection system with the passive anode 326 and the control unit 313. Should a fault occur in the active corrosion protection system, then the fifth switch 335 is switched from its open position to the closed position to connect the passive anode 326 to the cathode 317. Prior to this action, or at least at the same time, the first switch 331 is controlled to its open position to disconnect the active anode 315 from the battery 310 and the cathode 317. This electrical circuit provides a passive back-up corrosion protection system for the vessel. As indicated above, the control unit 313 is adapted to measure electrical potential of the cathode 317 with the reference electrode 324 as a ground reference. The electrical potential is indicative of the surface polarization at the interface between the cathode 317 and the water W. The control unit 313 is further adapted to control the voltage supplied by the DC power source 310 based at least partly on the electrical potential of the cathode 317 with the reference electrode 324 as a ground reference, as measured by the sensor 330. Through control of the voltage supplied by the DC power source 310, an electrical current I3 flowing between the passive anode 326 and the cathode 317 is controlled. Thus, the electrical current I3 runs through an electrical circuit comprising the passive anode 326, the cathode 317 and the electrolyte W during passive corrosion protection mode. As an alternative, the passive anode 326 may be separated from the power source 310 and thus fed from another power source (not shown in Figure 3D).

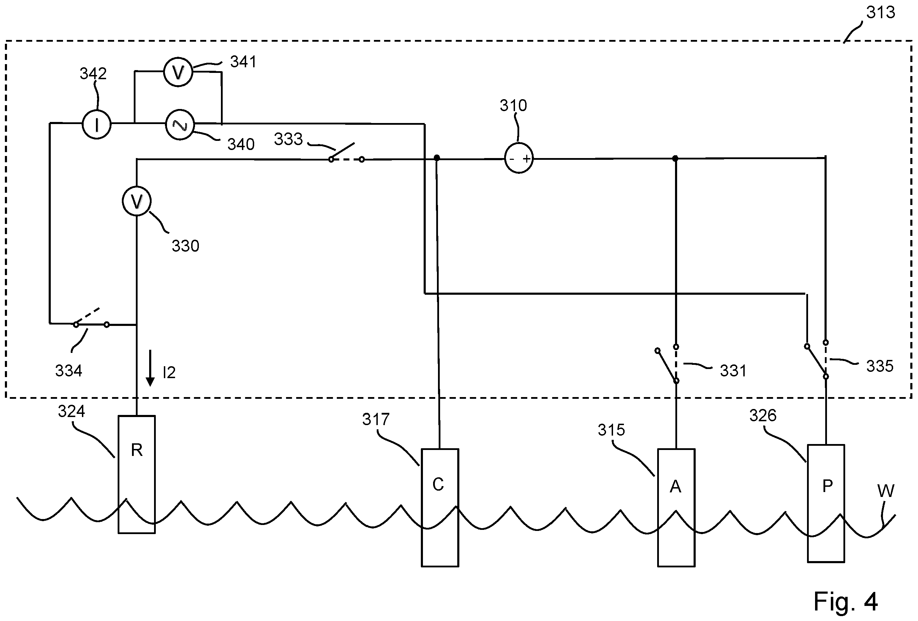

Figure 4 illustrates a further example for enabling salinity measurements in a scenario where a passive anode 326 indeed is available for connection to the corrosion protection system. The salinity measurement is in this example performed between the passive anode 326 and the reference electrode 324. As illustrated, in such example, the second switch 332 can be omitted, and the cathode 317 is thus fixedly connected to the negative terminal of the DC power source 310. Advantageously, even if the surface area of the passive anode 326 changes over time, it is still generally much larger than the reference electrode 324 and the resistance over the passive anode 326 would be negligible in comparison.

Similar to the example of Figure 3A, in active corrosion protection mode, the first switch 331 will be controlled to connect the active anode 315 to the positive terminal of the DC power source 310 and the cathode 317 while keeping the third switch 333 open. Each time the potential between the cathode 317 and the reference electrode 324 is to be measured using the voltage sensor 330 (e.g. every 10 seconds), the first switch 331 is opened (the fourth switch 334 is also open) while the third switch 333 is closed. As previously described, during active corrosion protection mode, the passive anode 326 is disconnected from the DC power source 310.

Further, similar to the example of Figure 3D, in passive corrosion protection mode, the first switch 331 will be controlled to open for disconnecting the active anode 315 from the positive terminal of the DC power source 310 while closing the fifth switch 335 to connect the passive anode 326 to the positive terminal of the DC power source 310.

However, as shown in Figure 4, and which differs from the previously described salinity measurement mode of Figure 3B, in this example the salinity measurement mode will be attained by connecting the passive anode 326 via the fifth switch 335 to the AC power source 340 and further via the fourth switch 334 to the reference electrode 324. At this stage, the first switch 331 must be opened (whereas in Figure 3B, it does not matter whether the first switch 331 is open or closed). Thus, a current I2 will flow through the path formed by the passive anode 326, the fifth switch 335, the fourth switch 334, the reference electrode 324 and the water W. The salinity measurement is undertaken as previously described utilizing voltage sensor 341 and current sensor 342. Further, as is understood, equations (1) and (2) are equally applicable in this example with the areas of the passive anode 326 and/or the reference electrode 324 taken into consideration.

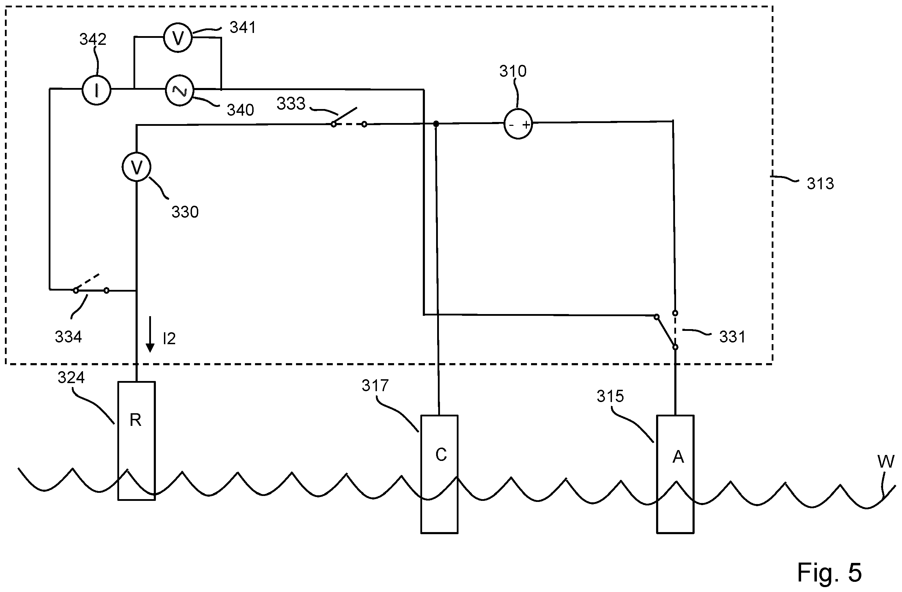

Figure 5 illustrates a further example for enabling salinity measurements. The salinity measurement is in this example performed between the active anode 315 and the reference electrode 324. As illustrated, in such example, the second switch 332 can be omitted. While the system of Figure 5 does not comprise a passive anode 326 for attaining passive corrosion protection, it is understood that such passive anode 326 may be connected to the DC power source 310 via the fifth switch 335 as previously has been described.

Similar to the example of Figure 3A, in active corrosion protection mode, the first switch 331 will be controlled to connect the active anode 315 to the positive terminal of the DC power source 310 and the cathode 317 while keeping the third switch 333 open. Each time the potential between the cathode 317 and the reference electrode 324 is to be measured using the voltage sensor 330 (e.g. every 10 seconds), the first switch 331 is opened (the fourth switch 334 is also open) while the third switch 333 is closed.

However, as shown in Figure 5, and which differs from the previously described salinity measurement modes of Figure 3B and 4, in this example the salinity measurement mode will be attained by connecting the active anode 315 via the first switch 331 to the AC power source 340 and further via the fourth switch 334 to the reference electrode 324 (while keeping the third switch 333 open). Thus, a current I2 will flow through the path formed by the active anode 315, the first switch 331, the fourth switch 334, the reference electrode 324 and the water W. The salinity measurement is undertaken as previously described utilizing voltage sensor 341 and current sensor 342. Further, as is understood, equations (1) and (2) are equally applicable in this example with the areas of the active anode 315 and/or the reference electrode 324 taken into consideration.

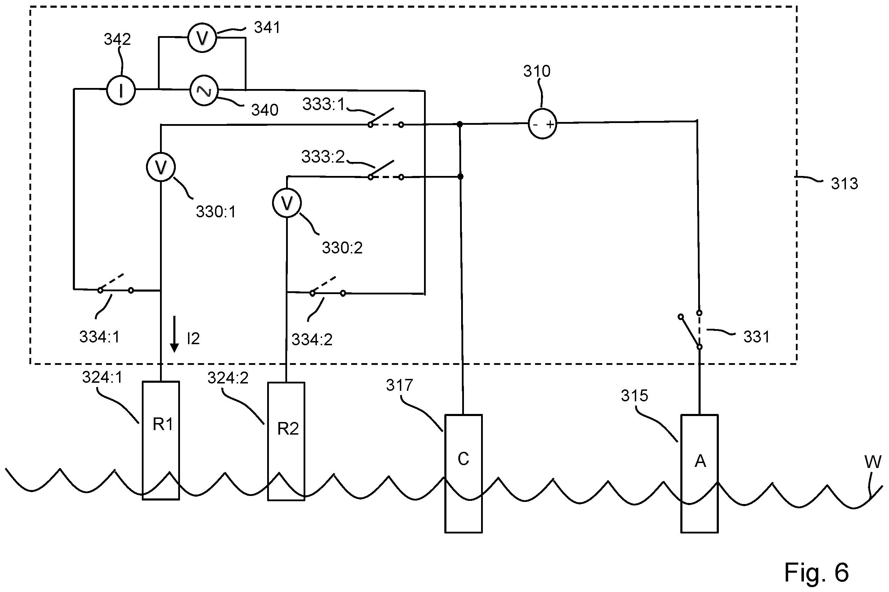

Figure 6 illustrates a further example for enabling salinity measurements. As previously mentioned, a plurality of reference electrodes may be utilized for redundancy purposes. Illustrated in Figure 6 are two reference electrodes 324:1 (R1) and 324:2 (R2), and the salinity measurement is in this example performed between the first reference electrode 324:1 and the second reference electrode 324:2. As illustrated, in such example, the second switch 332 can be omitted. While the system of Figure 6 does not comprise a passive anode 326 for attaining passive corrosion protection, it is understood that such passive anode 326 may be connected to the DC power source 310 via the fifth switch 335 as previously has been described. As is understood, while two reference electrodes 324:1, 324:2 are included in the circuit of Figure 6, further reference electrodes may be included in a particular implementation. In addition to the previously discussed advantages associated with using reference electrodes for salinity measurement, measuring the salinity between the reference electrodes 324:1, 324:2 further has the advantage that the distance between the two reference electrodes is constant and will provide the most precise and reliable measurement results.

Similar to the example of Figure 3A, in active corrosion protection mode, the first switch 331 will be controlled to connect the active anode 315 to the positive terminal of the DC power source 310 and the cathode 317 while keeping the third switches 333:1, 333:2 open (one for each reference electrode 324:1, 324:2). Each time the potential between the cathode 317 and either of the first reference electrode 324:1 and the second reference electrode 324:2 is to be measured using the corresponding voltage sensor 330:1 or 330:2 (e.g. every 10 seconds), the first switch 331 is opened (both of fourth switches 334:1, 334:2 are also open) while either of the third switches 333:1, 333:2 is closed depending on which of the first and second electrodes 324:1, 324:2 to be used.

However, as shown in Figure 6, and which differs from the previously described salinity measurement modes of Figure 3B, 4 and 5, in this example the salinity measurement mode will be attained by connecting the first reference electrode 324:1 via a first 334:1 of the fourth switches to the AC power source 340 and further via a second 334:2 of the fourth switches to the second reference electrode 324:2 (while keeping the third switches 333:1, 333:2 open as well as the first switch 331 open for disconnecting the active anode 315 from the DC power source 310). Thus, a current I2 will flow through the path formed by the first reference electrode 324:1, the first 334:1 of the fourth switches, the second 334:2 of the fourth switches, the second reference electrode 324:2 and the water W. The salinity measurement is undertaken as previously described utilizing voltage sensor 341 and current sensor 342. Further, as is understood, equations (1) and (2) are equally applicable in this example with the areas of one or both of the reference electrodes 324:1, 324:2 taken into consideration.

It should be noted that the electrical circuits described in the examples above are only some of a multitude of possible solutions allowing the circuit to be switched between a corrosion protection mode, a salinity measurement mode and a passive protection mode. Hence, the inventive concept is not limited to the electrical circuit shown in Figures 3A-D and 4-6.

Example 1: Arrangement for measuring salinity in water, which arrangement is part of an impressed current cathodic protection, ICCP, system having an electrical circuit comprising:

- a control unit (313) for controlling the impressed current cathodic protection system;
- a first direct current power source (310); a plurality of electrodes including:

  - at least one active anode electrode (315) connectable to a positive terminal of the first power source (310);
  - at least one cathode electrode (317) connectable to a negative terminal of the first power source (310); and
  - at least one reference electrode (324) connectable to said cathode electrode (317) for measuring an electrical potential of the cathode electrode (317) with the reference electrode (324) as a ground reference, the electrical potential being indicative of the surface polarization of the water at the cathode electrode (317), and the control unit (313) being configured to control the power supplied by the first direct current power source (310) based on the measured electrical potential; the electrical circuit further comprising:

  - a second power source (340), a first terminal to which the at least one reference electrode (324) is connectable;
  - a voltage sensor (341) detecting an output voltage impressed on the reference electrode (324) upon the reference electrode being connected to the second source of electric power (340);
  - a current sensor (342) detecting a current supplied to the reference electrode (324) upon the reference electrode being connected to the second source of electric power (340);

wherein
the control unit (313) is configured to initiate a measurement sequence at determined intervals, during which measurement sequence the control unit is configured to:

- disconnect the reference electrode (324) from the cathode electrode (317);
- connect the reference electrode (324) to the first terminal of the second power source (340);
- connect at least another one of the electrodes to a second terminal of the second power source (340) while disconnecting said another electrode from the first direct current power source (310);
- register said output voltage;
- register said current;
- determine the resistance of the circuit formed by the reference electrode (324) and said another one of the electrodes using the output voltage and the current; and
- calculate the resistivity of the water based on the determined circuit resistance and at least one stored electrode property value, which resistivity is inversely proportional to the salinity.

Example 2. The arrangement of example 1, said at least another one of the electrodes being the cathode electrode (317).

Example 3. The arrangement of example 1, said at least another one of the electrodes being the active anode electrode (317).

Example 4. The arrangement of example 1, the at least one reference electrode comprising at least two reference electrodes (324:1, 324:2) each individually connectable to said cathode electrode (317), said at least another one of the electrodes being a second one (324:2) of the at least two reference electrodes.

Example 5. The arrangement of any one of the preceding examples, the plurality of electrodes further including: at least one passive anode electrode (326) connectable to the positive terminal of the first direct current power source (310), the passive anode electrode (326) serving as a sacrificial electrode for back-up protection, the control unit (313) being configured to connect the passive anode electrode (326) to the positive terminal of the first direct current power source (310), while disconnecting the active anode electrode (315) from the positive terminal of the first direct current power source (310), to form a circuit with the cathode electrode (317) to attain back-up protection.

Example 6. The arrangement of example 1, the plurality of electrodes further including: at least one passive anode electrode (326) connectable to the positive terminal of the first direct current power source (310), the passive anode electrode (326) serving as a sacrificial electrode for back-up protection, the control unit (313) being configured to connect the passive anode electrode (326) to the positive terminal of the first direct current power source (310), while disconnecting the active anode electrode (315) from the positive terminal of the first direct current power source (310), to form a circuit with the cathode electrode (317) to attain back-up protection, said at least another one of the electrodes being the passive anode electrode (326).

Example 7. The arrangement of any one of the preceding examples, the second power source (340) being an alternative current power source or a direct current power source;

Example 8. The arrangement of any one of the preceding examples, the control unit (313) being configured to disconnect the active anode electrode (315) from the first direct current power source (310) upon said another of the electrodes being connected to the second power source (340).

Example 9. The arrangement of any one of the preceding examples, wherein the at least one stored electrode property value is the surface area of said at least another one of the electrodes and/or the surface area of the reference electrode (324).

Example 10. The arrangement of any one of the preceding examples, wherein the control unit (313) is configured to maintain the impressed current cathodic protection system in operation if the determined resistivity is above a set threshold value.

Example 11. The arrangement of any one of the preceding examples, wherein the control unit (313) is configured to determine a current salinity value based on the determined resistivity and to generate an output signal indicating the salinity value to a user.

Example 12 The arrangement of any one of the preceding examples, wherein the control unit (313) is configured to monitor changes in the determined resistivity; to compare an increase in the determined resistivity to stored values for resistivity; and to determine if the increase is indicative of an electrical circuit malfunction.

Example 13. A marine object (100) provided with an impressed current cathodic protection system comprising an arrangement for measuring salinity according to any one of the preceding examples.

Example 14. Method for measuring salinity in water using an arrangement being part of an impressed current cathodic protection, ICCP, system having an electrical circuit comprising:

- a control unit (313) for controlling the impressed current cathodic protection system
- a first direct current power source (310); a plurality of electrodes including:

  - at least one active anode electrode (315) connectable to a positive terminal of the first power source (310);
  - at least one cathode electrode (317) connectable to a negative terminal of the first power source (310); and
  - at least one reference electrode (324) connectable to said cathode electrode (317) for measuring an electrical potential of the cathode electrode (317) with the reference electrode (324) as a ground reference, the electrical potential being indicative of the surface polarization of the water at the cathode electrode (317), and the control unit (313) being configured to control the power supplied by the first direct current power source (310) based on the measured electrical potential; the electrical circuit further comprising:

    - a second power source (340), a first terminal to which the at least one reference electrode (324) is connectable;
    - a voltage sensor (341) detecting an output voltage impressed on the reference electrode (324) upon the reference electrode being connected to the second source of electric power (340);
    - a current sensor (342) detecting a current supplied to the reference electrode (324) upon the reference electrode being connected to the second source of electric power (340);

wherein

the control unit (313) is configured to initiate a measurement sequence at determined intervals, during which measurement sequence the control unit is configured to:

- disconnect (S101) the reference electrode (324) from the cathode electrode (317);
- connect (S102) the reference electrode (324) to the first terminal of the second power source (340);
- connect (S103) at least another one of the electrodes to a second terminal of the second power source (340) while disconnecting said another electrode from the first direct current power source (310);
- register (S104) said output voltage;
- register (S105) said current;
- determine (S106) the resistance of the circuit formed by the reference electrode (324) and said another one of the electrodes using the output voltage and the current; and
- calculate (S107) the resistivity of the water based on the determined circuit resistance and at least one stored electrode property value, which resistivity is inversely proportional to the salinity.

Example 15. The method of example 14, said at least another one of the electrodes being the cathode electrode (317).

Example 16. The method of example 14, said at least another one of the electrodes being the active anode electrode (317).

Example 17. The method of example 14, the at least one reference electrode comprising at least two reference electrodes (324:1, 324:2) each individually connectable to said cathode electrode (317), said at least another one of the electrodes being a second one (324:2) of the at least two reference electrodes.

Example 18. The method of example 14, the plurality of electrodes further including: at least one passive anode electrode (326) connectable to the positive terminal of the first direct current power source (310), the passive anode electrode (326) serving as a sacrificial electrode for back-up protection, the control unit (313) being configured to connect the passive anode electrode (326) to the positive terminal of the first direct current power source (310), while disconnecting the active anode electrode (315) from the positive terminal of the first direct current power source (310), to form a circuit with the cathode electrode (317) to attain back-up protection, said at least another one of the electrodes being the passive anode electrode (326).

Example 19. A computer program product (351) comprising program code (350) for performing, when executed by the control circuit (313), the method of any of examples 14-18.

Example 20. A non-transitory computer-readable storage medium (351) comprising instructions, which when executed by the control circuit (313) cause the arrangement to perform the method of any of examples 14-18.

The terminology used herein is for the purpose of describing particular aspects only and is not intended to be limiting of the disclosure. As used herein, the singular forms "a," "an," and "the" are intended to include the plural forms as well, unless the context clearly indicates otherwise. As used herein, the term "and/or" includes any and all combinations of one or more of the associated listed items. It will be further understood that the terms "comprises," "comprising," "includes," and/or "including" when used herein specify the presence of stated features, integers, actions, steps, operations, elements, and/or components, but do not preclude the presence or addition of one or more other features, integers, actions, steps, operations, elements, components, and/or groups thereof.

It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

It is to be understood that the present disclosure is not limited to the aspects described above and illustrated in the drawings; rather, the skilled person will recognize that many changes and modifications may be made within the scope of the present disclosure and appended claims. In the drawings and specification, there have been disclosed aspects for purposes of illustration only and not for purposes of limitation, the scope of the disclosure being set forth in the following claims.

## Claims

1. Arrangement for measuring salinity in water, which arrangement is part of an impressed current cathodic protection, ICCP, system having an electrical circuit comprising:

   - a control unit (313) for controlling the impressed current cathodic protection system;
   - a first direct current power source (310); a plurality of electrodes including:

     - at least one active anode electrode (315) connectable to a positive terminal of the first power source (310);
     - at least one cathode electrode (317) connectable to a negative terminal of the first power source (310); and
     - at least one reference electrode (324) connectable to said cathode electrode (317) for measuring an electrical potential of the cathode electrode (317) with the reference electrode (324) as a ground reference, the electrical potential being indicative of the surface polarization of the water at the cathode electrode (317), and the control unit (313) being configured to control the power supplied by the first direct current power source (310) based on the measured electrical potential; the electrical circuit further comprising:

       - a second power source (340), a first terminal to which the at least one reference electrode (324) is connectable;
       - a voltage sensor (341) detecting an output voltage impressed on the reference electrode (324) upon the reference electrode being connected to the second source of electric power (340);
       - a current sensor (342) detecting a current supplied to the reference electrode (324) upon the reference electrode being connected to the second source of electric power (340);

   wherein

   the control unit (313) is configured to initiate a measurement sequence at determined intervals, during which measurement sequence the control unit is configured to:

   - disconnect the reference electrode (324) from the cathode electrode (317);

   - connect the reference electrode (324) to the first terminal of the second power source (340);
   - connect at least another one of the electrodes to a second terminal of the second power source (340) while disconnecting said another electrode from the first direct current power source (310);
   - register said output voltage;
   - register said current;
   - determine the resistance of the circuit formed by the reference electrode (324) and said another one of the electrodes using the output voltage and the current; and
   - calculate the resistivity of the water based on the determined circuit resistance and at least one stored electrode property value, which resistivity is inversely proportional to the salinity.

2. The arrangement of claim 1, said at least another one of the electrodes being the cathode electrode (317).

3. The arrangement of claim 1, said at least another one of the electrodes being the active anode electrode (317).

4. The arrangement of claim 1, the at least one reference electrode comprising at least two reference electrodes (324:1, 324:2) each individually connectable to said cathode electrode (317), said at least another one of the electrodes being a second one (324:2) of the at least two reference electrodes.

5. The arrangement of any one of the preceding claims, the plurality of electrodes further including: at least one passive anode electrode (326) connectable to the positive terminal of the first direct current power source (310), the passive anode electrode (326) serving as a sacrificial electrode for back-up protection, the control unit (313) being configured to connect the passive anode electrode (326) to the positive terminal of the first direct current power source (310), while disconnecting the active anode electrode (315) from the positive terminal of the first direct current power source (310), to form a circuit with the cathode electrode (317) to attain back-up protection.

6. The arrangement of claim 1, the plurality of electrodes further including: at least one passive anode electrode (326) connectable to the positive terminal of the first direct current power source (310), the passive anode electrode (326) serving as a sacrificial electrode for back-up protection, the control unit (313) being configured to connect the passive anode electrode (326) to the positive terminal of the first direct current power source (310), while disconnecting the active anode electrode (315) from the positive terminal of the first direct current power source (310), to form a circuit with the cathode electrode (317) to attain back-up protection, said at least another one of the electrodes being the passive anode electrode (326).

7. The arrangement of any one of the preceding claims, the second power source (340) being an alternative current power source or a direct current power source;

8. The arrangement of any one of the preceding claims, the control unit (313) being configured to disconnect the active anode electrode (315) from the first direct current power source (310) upon said another of the electrodes being connected to the second power source (340).

9. The arrangement of any one of the preceding claims, wherein the at least one stored electrode property value is the surface area of said at least another one of the electrodes and/or the surface area of the reference electrode (324).

10. The arrangement of any one of the preceding claims, wherein the control unit (313) is configured to maintain the impressed current cathodic protection system in operation if the determined resistivity is above a set threshold value.

11. The arrangement of any one of the preceding claims, wherein the control unit (313) is configured to determine a current salinity value based on the determined resistivity and to generate an output signal indicating the salinity value to a user.

12. A marine object (100) provided with an impressed current cathodic protection system comprising an arrangement for measuring salinity according to any one of the preceding claims.

13. Method for measuring salinity in water using an arrangement being part of an impressed current cathodic protection, ICCP, system having an electrical circuit comprising:

- a control unit (313) for controlling the impressed current cathodic protection system
- a first direct current power source (310); a plurality of electrodes including:

- at least one active anode electrode (315) connectable to a positive terminal of the first power source (310);
- at least one cathode electrode (317) connectable to a negative terminal of the first power source (310); and
- at least one reference electrode (324) connectable to said cathode electrode (317) for measuring an electrical potential of the cathode electrode (317) with the reference electrode (324) as a ground reference, the electrical potential being indicative of the surface polarization of the water at the cathode electrode (317), and the control unit (313) being configured to control the power supplied by the first direct current power source (310) based on the measured electrical potential; the electrical circuit further comprising:

- a second power source (340), a first terminal to which the at least one reference electrode (324) is connectable;
- a voltage sensor (341) detecting an output voltage impressed on the reference electrode (324) upon the reference electrode being connected to the second source of electric power (340);
- a current sensor (342) detecting a current supplied to the reference electrode (324) upon the reference electrode being connected to the second source of electric power (340);

wherein
the control unit (313) is configured to initiate a measurement sequence at determined intervals, during which measurement sequence the control unit is configured to:

- disconnect (S101) the reference electrode (324) from the cathode electrode (317);
- connect (S102) the reference electrode (324) to the first terminal of the second power source (340);
- connect (S103) at least another one of the electrodes to a second terminal of the second power source (340) while disconnecting said another electrode from the first direct current power source (310);
- register (S104) said output voltage;
- register (S105) said current;
- determine (S106) the resistance of the circuit formed by the reference electrode (324) and said another one of the electrodes using the output voltage and the current; and
- calculate (S107) the resistivity of the water based on the determined circuit resistance and at least one stored electrode property value, which resistivity is inversely proportional to the salinity.

14. A computer program product (351) comprising program code (350) for performing, when executed by the control circuit (313), the method of claim 13.

**15.** A non-transitory computer-readable storage medium (351) comprising instructions, which when executed by the control circuit (313) cause the arrangement to perform the method of claim 13.

Fig. 1

Fig. 2

Fig. 3A

Fig. 3B

EP 4 624 629 A1

Fig. 3C

Fig. 3D

Fig. 4

EP 4 624 629 A1

Fig. 5

Fig. 6

Europäisches
Patentamt

European
Patent Office

Office européen
des brevets

# EUROPEAN SEARCH REPORT

**Application Number**

EP 24 16 6461

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/096293 A1 (RÄFTEGÅRD VIKTOR [SE]) 30 March 2023 (2023-03-30) * the whole document * | 1-15 | INV. C23F13/02 C23F13/06 |
| A | US 2022/349065 A1 (RÄFTEGÅRD VIKTOR [SE]) 3 November 2022 (2022-11-03) * the whole document * | 1-15 | |
| A | US 4 528 460 A (STAERZL RICHARD E [US]) 9 July 1985 (1985-07-09) * the whole document * | 1-15 | |
| A | US 2020/216966 A1 (HASHEMI FARZAD [CA]) 9 July 2020 (2020-07-09) * the whole document * | 1-15 | |

**TECHNICAL FIELDS SEARCHED (IPC)**

C23F

The present search report has been drawn up for all claims

| Place of search | Date of completion of the search | Examiner |
|---|---|---|
| Munich | 26 September 2024 | Leu, Oana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 6461

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2024

| Patent document cited in search report | | Publication date | Patent family member(s) | | Publication date |
|---|---|---|---|---|---|
| US 2023096293 | A1 | 30-03-2023 | CN | 116219442 A | 06-06-2023 |
| | | | EP | 4190942 A1 | 07-06-2023 |
| | | | US | 2023096293 A1 | 30-03-2023 |
| US 2022349065 | A1 | 03-11-2022 | CN | 114450435 A | 06-05-2022 |
| | | | EP | 4038219 A1 | 10-08-2022 |
| | | | US | 2022349065 A1 | 03-11-2022 |
| | | | WO | 2021063507 A1 | 08-04-2021 |
| US 4528460 | A | 09-07-1985 | CA | 1213562 A | 04-11-1986 |
| | | | GB | 2133592 A | 25-07-1984 |
| | | | JP | S6324075 B2 | 19-05-1988 |
| | | | JP | S59177363 A | 08-10-1984 |
| | | | US | 4528460 A | 09-07-1985 |
| US 2020216966 | A1 | 09-07-2020 | CA | 3061869 A1 | 08-11-2018 |
| | | | US | 2020216966 A1 | 09-07-2020 |
| | | | US | 2024052500 A1 | 15-02-2024 |
| | | | WO | 2018203221 A1 | 08-11-2018 |

EPO FORM P0459

For more details about this annex : see Official Journal of the European Patent Office, No. 12/82